# EUROPEAN PATENT APPLICATION

(11) **EP 2 484 312 A1**
(43) Date of publication of application: **08.08.2012**
(21) Application number: 10820510.5
(22) Date of filing: 28.09.2010
(51) Int. Cl.: A61F 2/28, A61B 17/56, A61M 5/142

(54) **MEDICAL AGENT INFUSION DEVICE**

(30) Priority: 29.09.2009 JP 2009224687
(71) Applicant: Terumo Kabushiki Kaisha, Tokyo 151-0072 (JP)
(72) Inventor: MATSUMOTO Atsushi, Ashigarakami-gun Kanagawa 259-0151 (JP); SARUHASHI Makoto, Ashigarakami-gun Kanagawa 259-0151 (JP)
(74) Representative: TBK
(86) International application number: PCT/JP2010/066795
(87) International publication number: WO 2011/040395

(57) **Abstract**

A medicine injection device (10) is provided with a medicine ejection unit (14) having a flow-in port (31) of liquid (19) on the proximal side of a tube body (24), and a pressure generation unit (16) which includes a pressure generating chamber (18) for generating pressure acting upon a working chamber (30) of the medicine ejection unit (14) through the liquid (19) and which ejects medicine from an ejection port (20) by making a floating gasket (26) of the medicine ejection unit (14) progress depending on aforesaid pressure. The pressure generation unit (16) includes a pressure portion cylinder (34) provided inside the pressure generating chamber (18), a first pusher (38) provided with a hollow gasket (36) for defining the inner volume of the pressure generating chamber (18) by sliding inside the pressure portion cylinder (34) in a liquid-tight state and a second pusher (40) which generates the pressure inside the pressure generating chamber (18) by pressing a distal end head portion (40c) into the inside of the pressure generating chamber (18), and the pressure portion cylinder (34) and the first pusher (38) are coupled through an engagement mechanism (42) in which fixation and fixation release are possible for each other for every predetermined pitch in the axial line direction thereof.

## Description

### TECHNICAL FIELD

The present invention relates to a medicine injection device for ejecting a medicine from a medicine ejection unit by pressure generated in a pressure generation unit and for injecting such medicine into a space to be injected.

### BACKGROUND ART

In recent years, there has been carried out, with respect to compression fracture of a vertebral body caused by osteoporosis or cancer, percutaneous vertebroplasty (PVP) injecting a filling member which is hardened along with the time elapsing after a bone biopsy needle is stuck into a bone fractured vertebral body. For this filling member, for example, there has been used calcium phosphate based bone cement, polymethyl methacrylate bone cement or the like having an X-ray contrast property (hereinafter, simply referred to also as "bone cement"). Usually, the bone cement has a very high viscosity and also, the inside of the vertebra which is filled with the bone cement has a very large pressure drop caused by a spongy substance or the like, so that during injection, it is necessary to inject the cement little by little with a high pressure of 3MPa or more.

Therefore, for such an injection device, a constitution is used in which the bone cement is ejected while being applied with a high pressure by a general piston type or feed screw type syringe. On the other hand, a constitution has been proposed in which the bone cement is injected while suppressing the pressure to a certain degree by lowering the amount of injection gradually (see Japanese unexamined PCT patent publication No. 2004-507312).

### DISCLOSURE OF THE INVENTION

As described above, for example, with respect to the medicine injection device used for injecting a liquid medicine (bone cement) having high viscosity into a space which has a high pressure drop such as the inside of the bone, it is desirable to employ a constitution in which the medicine can be ejected with high pressure by a proper amount every time.

However, it is difficult for an injection device of a usual piston type to generate an adequate high pressure and to inject medicine by a proper amount for every time depending on the generated high pressure. Also, it becomes possible for a feed screw type injection device to employ quantitative injection at a high pressure, but the high pressure state inside the cylinder is not released and is always maintained during the injection, so that in case of desiring to stop the injecting operation, it is difficult to stop it immediately. Further, in a constitution of injecting bone cement while suppressing the pressure to a certain degree by decreasing the amount of injection gradually such as a constitution described in Japanese unexamined PCT patent publication No. 2004-507312, there is a possibility that burden to a patient increases caused by the fact that the necessary time period until the injection completion lengthens and in addition, there is also a possibility that it is not possible to inject the necessary amount completely caused by the low pressure injection depending on the condition of the vertebral body or the like.

The present invention was invented in consideration of such a problem and is addressed to provide a medicine injection device in which it is possible to inject or eject the medicine with a predetermined high pressure by a proper amount for every time when injecting the medicine into the inside of a space to be injected and concurrently, in which the pressure inside the cylinder is easily releasable and the injection operation becomes easy-to-use.

A medicine injection device relating to the present invention is provided with a medicine ejection unit including a flow-in port of fluid on the proximal side of a tube body in which the inside of the tube body is partitioned by a sub gasket slidable in a liquid-tight state into a working chamber on the flow-in side of the fluid and an action chamber on the distal side, and a pressure generation unit including a pressure generating chamber which generates pressure acting upon the working chamber through the fluid in which medicine is ejected by making the sub gasket progress by the pressure, characterized in that the pressure generation unit includes a pressure portion cylinder provided inside the pressure generating chamber, a first pusher provided with a main gasket for defining the inner volume of the pressure generating chamber by sliding inside the pressure portion cylinder in a liquid-tight state, and a second pusher which passes-through the inside of the first pusher and concurrently, which is slidable in a liquid-tight state in a slide hole penetratingly formed in the slide direction of the main gasket and which generates the pressure inside the pressure generating chamber by pressing a distal end head portion into the pressure generating chamber; and the pressure portion cylinder and the first pusher are coupled through an engagement mechanism in which fixation and fixation release are possible for each other for every predetermined pitch in the axial line direction thereof.

According to such a constitution, by sliding the slide hole of the main gasket and pressing the second pusher thereinto in a state of fixing both the pressure portion cylinder and the first pusher in the axial line direction, it is possible to generate a high pressure through aforesaid fluid in the pressure generating chamber and it is possible to make the sub gasket progress by the generated pressure. Thus, it is possible to eject the medicine with a high pressure from the medicine ejection unit by a proper amount for every time and to inject the medicine into the inside of the non-injection space. Also, after the ejection, by making the main gasket progress inside the pressure generating chamber by shortening the space between the pressure portion cylinder and the first pusher through the engagement mechanism, it is possible for the second pusher to return to the original position and the pressure inside the system is reliably released simultaneously with the completion of the preparation of the next ejection process. Therefore, it is possible to prevent the pressure, which is generated inside the medicine ejection unit or inside the pressure generation unit by the pressing-in of the second pusher, from acting on a steady basis and it is possible to prevent erroneous medicine ejection on an occasion such as operation standby or the like.

Note that when the region from the working chamber to the pressure generating chamber is filled with an incompressible fluid which is substantially not compressed by the pressure generated in the pressure generation unit, it is possible to drive the sub gasket without substantially losing the pressure generated in the pressure generation unit.

In this case, when the pressure portion cylinder communicates with the working chamber at the distal side thereof and concurrently, includes, at the proximal side thereof, a tubular portion formed with an opening end through which the main gasket is inserted; and the first pusher includes a first tubular portion on which the main gasket is fixed at the distal side thereof and which is insertable into the tubular portion, and a second tubular portion forming a tubular space into which the tubular portion is insertable with respect to the first tubular portion by being provided coaxially by sandwiching a predetermined space in the outer diameter direction of the first tubular portion, it is possible for the pressure portion cylinder to be inserted into the inside of the first pusher and to be coupled therewith easily.

When the engagement mechanism includes a first rib which is provided on the outer circumferential surface of the tubular portion of the pressure portion cylinder and which projects from a portion in the circumferential direction of the outer circumferential surface toward the outer diameter direction, and second ribs which are provided by a plurality of numbers and by having a predetermined pitch in the axial direction of the second tubular portion on an inner circumferential surface of the second tubular portion of the first pusher and which are engageable with the first rib for every pitch of the predetermined pitch in the axial direction of the second tubular portion by projecting from the portion in the circumferential direction of the inner circumferential surface toward the tubular space; and there is formed, in the tubular space, a rib path in which the first rib is passable on the side of the second rib toward the axial direction of the second tubular portion, it is possible to realize the fixation and fixation release in the axial direction between the pressure portion cylinder and the first pusher by a simple constitution.

When the first rib is a plate-shaped member which is diameter-expanded for a portion of the outer circumferential surface of the tubular portion toward the outer diameter direction thereof and concurrently, is provided by at least one pair facing to each other in the diameter direction of the tubular portion; the second rib is a plate-shaped member which is diameter-reduced for a portion of the inner circumferential surface of the second tubular portion toward the inner diameter direction thereof and concurrently, is provided by at least one pair facing to each other in the diameter direction of the second tubular portion; and the rib path is formed between the facing second ribs, it is possible to engage the first rib and the second rib at two places facing each other in the circumferential direction, so that it is possible to fix the pressure portion cylinder and the first pusher further tightly compared with a case, for example, in which each rib is installed by only one piece in the circumferential direction, and also endurance on an occasion of the pressure generation is improved.

It is allowed to employ a configuration in which there is provided, for the second rib, a rib set by one pair facing to each other in the diameter direction of the second tubular portion in which a first small rib and a second small rib which are formed to be shorter than the circumferential direction length of the first rib are aligned in the circumferential direction of the inner circumferential surface of the second tubular portion; the first small ribs alone and the second small ribs alone are respectively arranged to face each other between the facing rib sets; and in the axial direction of the second tubular portion, the pitch between the first small ribs alone and the pitch between the second small ribs alone are respectively set to be the predetermined pitches with which the first rib is engageable, and the pitches between the first small ribs and the second small ribs are set to be half the predetermined pitches. Thus, by rotating the pressure portion cylinder clockwise and counterclockwise alternately with respect to the first pusher, it is possible, while the first rib engages with respect to the first small rib and second small rib alternately, to easily fix and move the pressure portion cylinder and the first pusher by an amount of aforesaid half pitch.

In this case, when there is formed, at both end portions of the first rib in the circumferential direction of the tubular portion, a first inclination surface whose width narrows toward the proximal side in the axial direction of the tubular portion; and there is formed, at an end portion facing to the rib path of the first small rib and the second small rib which constitute the rib set, a second inclination surface whose width narrows toward the distal side in the axial direction of the second tubular portion and which the first inclination surface can get over by sliding, it becomes possible to carry out the fixation and movement of the pressure portion cylinder and first pusher by the half pitches further smoothly.

When the first rib has a predetermined pitch and is provided by a plurality of numbers in the axial direction of the second tubular portion, and the respective first ribs are engageable with the respective second ribs simultaneously, the engagement strength, the stability on an occasion of the engagement or the like between the pressure portion cylinder and the first pusher improve furthermore.

Note that it is advisable to employ such a constitution in which there is included a medicine ejection port in the action chamber of the tube body and the medicine is ejected from the medicine ejection port.

Also, if there is included a medicine container connection portion at the distal end portion of the tube body, and the sub gasket includes a plunger enterable into the inside of a medicine container which is connected to the action chamber side, it is possible to replace the medicine container easily and it is possible to improve the versatility and the usability of the medicine injection device furthermore.

When there is included a flexible tube which provides communication between the working chamber of the medicine ejection unit and the pressure generating chamber of the pressure generation unit, and the insides of the working chamber, the pressure generating chamber and the flexible tube are filled with the incompressible fluid, it becomes possible, for example, in case of carrying out medicine injection into the inside of a bone under X-ray illumination or the like, to carry out injection operation by installing the pressure generation unit at a position apart from the medicine ejection unit which is installed near an X-ray tube.

Note that the medicine injection device can be preferably used also in a case in which the medicine is bone cement to be injected into the inside of the bone. This is because it is necessary for the bone cement to be injected little by little into the bone at a predetermined high pressure, since bone cement has usually very high viscosity and also there is a very large pressure drop inside the bone due to a spongy substance or the like.

According to the present invention, by sliding the slide hole of the main gasket and pressing the second pusher thereinto in a state of fixing the pressure portion cylinder and the first pusher each other in the axial direction, it is possible to generate a high pressure through aforesaid fluid in the pressure generating chamber and to make the sub gasket progress. Consequently, it is possible to eject the medicine with a high pressure from the medicine ejection unit by a proper amount for every time and to inject the medicine into the inside of the non-injection space.

Also, after the ejection, by making the main gasket inside the pressure generating chamber progress by shortening the space between the pressure portion cylinder and the first pusher through the engagement mechanism, the second pusher is returned to the original position and the pressure inside the system is reliably released simultaneously with the completion of the preparation of the next ejection process. Therefore, it is possible to prevent the pressure, which is generated inside the medicine ejection unit or inside the pressure generation unit by the pressing-in of the second pusher, from acting on a steady basis and it is possible to prevent an erroneous medicine ejection on an occasion such as operation standby or the like.

### BRIEF DESCRIPTION OF DRAWINGS

FIG. 1 is a perspective view showing a constitution of a medicine injection device relating to a first exemplified embodiment of the present invention;
FIG. 2 is a partially cross-sectional side view of the medicine injection device shown in FIG. 1;
FIG. 3 is a cross-sectional view of the exploded side in which a main portion of a pressure generation unit constituting the medicine injection device shown in FIG. 1 is magnified;
FIG. 4 is a side cross-sectional view showing a state in which respective elements of the pressure generation unit are assembled from the state shown in FIG. 3;
FIG. 5 is a cross-sectional view along a V-V line in FIG. 3;
FIG. 6 is a cross-sectional view along a VI-VI line in FIG. 3;
FIG. 7A is a cross-sectional view along a VII-VII line in FIG. 4 in a state in which a first rib is arranged in a rib path;
FIG. 7B is a cross-sectional view in a state in which the first rib is engaged with a rib set by rotating a pressure portion cylinder from the state shown in FIG. 7A;
FIG. 8 is an operation flow relating to a medicine ejection by the medicine injection device shown in FIG. 1;
FIG. 9 is a cross-sectional view of the exploded side in which a main portion of a pressure generation unit constituting the medicine injection device relating to a second exemplified embodiment of the present invention is magnified;
FIG. 10 is a cross-sectional view along an X-X line in FIG. 9 in a state in which the pressure portion cylinder is inserted into the inside of a tubular space of a first pusher and the first rib is arranged in the rib path;
FIG. 11A is a cross-sectional view in a state in which the pressure portion cylinder is rotated counterclockwise from the state shown in FIG. 10;
FIG. 11B is a cross-sectional view in a state in which the pressure portion cylinder is rotated clockwise from the state shown in FIG. 11A;
FIG. 12 is a schematically explanatory view in which the pressure portion cylinder and the first pusher shown in FIG. 10 are developed by 360° in the circumferential direction in order to explain an engagement operation between the first rib and the rib set in the medicine injection device shown in FIG. 9; and
FIG. 13 is a partially omitted cross-sectional side view showing a medicine ejection unit 120 relating to a modification example.

### BEST MODE FOR CARRYING OUT THE INVENTION

Hereinafter, it will be explained with respect to medicine injection devices relating to the present invention by citing preferred exemplified embodiments thereof with reference to the attached drawings.

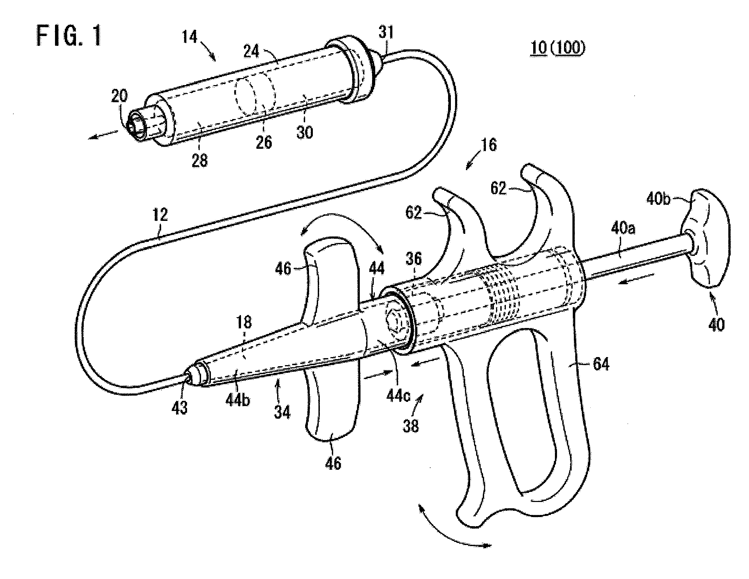

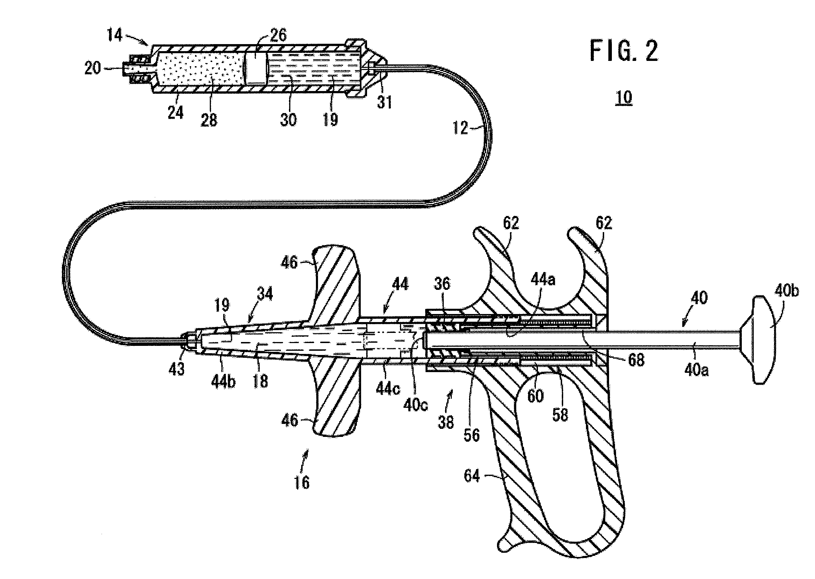

FIG. 1 is a perspective view showing a constitution of a medicine injection device 10 relating to a first exemplified embodiment of the present invention, and FIG. 2 is a partially cross-sectional side view of the medicine injection device 10 shown in FIG. 1. The medicine injection device 10 relating to this exemplified embodiment is provided with a medicine ejection unit 14 coupled to the distal side of a long flexible tube 12 and a pressure generation unit 16 coupled to the proximal side of the flexible tube 12, and it is an instrument for ejecting a predetermined medicine (filling member, injection member) from the medicine ejection unit 14 by pressure generated in a pressure generating chamber 18 of a pressure generation unit 16 through a predetermined liquid 19 and for injecting it into a desired space to be injected and is, for example, used for injecting bone cement into a bone in percutaneous vertebroplasty. As aforesaid medicine, for example, it is possible to use a bone cement such as calcium phosphate based bone cement (CPC), polymethyl methacrylate (PMMA) based bone cement or the like, and further, it is possible to use granules formed of an inorganic material such as calcium phosphate based ceramics, alumina ceramics, zirconia ceramics, titanium or the like.

Note that, in FIG. 1 and FIG. 2, the medicine ejection unit 14 side of the flexible tube 12 is referred to as "distal end" side, the pressure generation unit 16 side of the flexible tube 12 is referred to as "proximal end (rear end)" side, and this is similarly employed with respect to other drawings.

The medicine ejection unit 14 has a tubular body (cylindrical body) 24 provided with an ejection port (medicine ejection port) 20 at the distal end thereof, and a floating gasket (sub gasket) 26 which is slidable in a liquid-tight state inside aforesaid tube body 24, and the inside of the tube body 24 is partitioned by the floating gasket 26 into an action chamber 28 located on the distal side in the axial line direction and a working chamber 30 located on the proximal side and serving as the flow-in side of the liquid 19. The ejection port 20 is a luer connector and, when a medicine is injected into a bone, a needle or the like for bone cement, which is not shown, can be coupled thereto.

By being formed between the ejection port 20 and the floating gasket 26, the action chamber 28 is able to store aforesaid medicine and supplies the medicine to the ejection port 20 along with progression in the direction of the distal end of the floating gasket 26.

The working chamber 30 communicates with the flexible tube 12 by a flow-in port 31 of a liquid 19, which is formed on the proximal side of the tube body 24. Thus, the pressure generated through aforesaid liquid 19 at the pressure generating chamber 18 of the pressure generation unit 16 acts to the working chamber 30 through a flexible tube 12 and makes the floating gasket 26 progress. More specifically, by the flowing of the liquid 19, which fills the insides of the working chamber 30, the flexible tube 12 and the pressure generating chamber 18, from the pressure generating chamber 18 through the flexible tube 12 into the working chamber 30 by way of the flow-in port 31, the floating gasket 26 is made to progress. Therefore, the liquid 19 is made to be an incompressible fluid (for example, water) such that it serves as a motive force with which to make the floating gasket 26 progress without losing the pressure generated at the pressure generating chamber 18 as much as possible, and gaseous components with high compression ratios, such as air and the like, are excluded therefrom as much as possible.

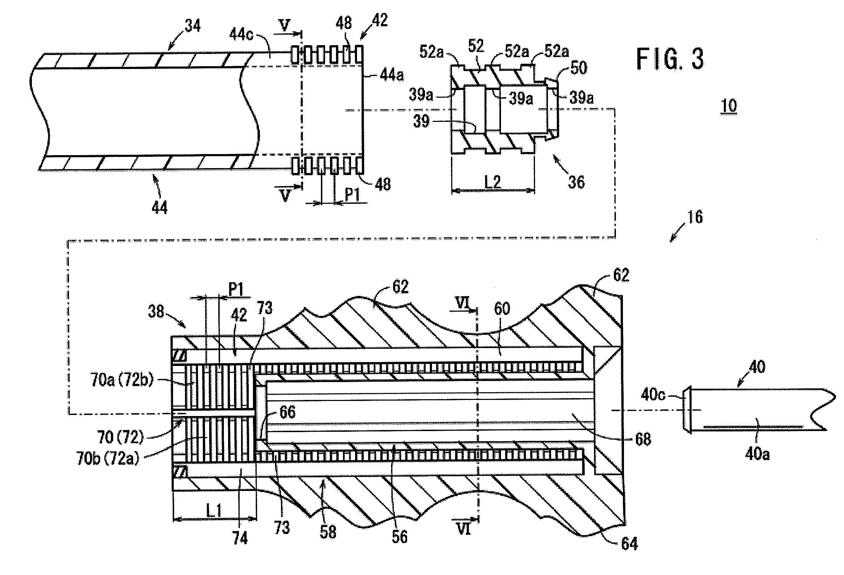

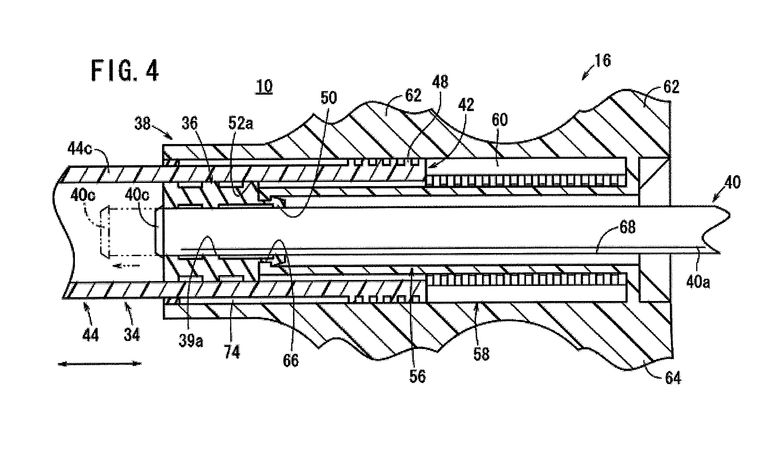

The pressure generation unit 16 includes a pressure portion cylinder 34 coupled with the flexible tube 12 at the distal end thereof, a hollow gasket (main gasket) 36 which defines the inner volume of the pressure generating chamber 18 by sliding in a liquid-tight state inside the pressure portion cylinder 34, a first pusher (first plunger) 38 having a double tube structure, to which the hollow gasket 36 is fixed on the distal side thereof, and a second pusher (second plunger) 40 provided with a rod 40a which passes-through the inside of the first pusher 38 from the proximal side to the distal side and which slides in a liquid-tight state with respect to a slide hole 39 (see FIG. 3 and FIG. 4) formed to pass-through in the slide direction of the hollow gasket 36. For the pressure portion cylinder 34 and the first pusher 38, the coupling position thereof in the axial direction is changeable appropriately by means of an engagement mechanism 42 (see FIG. 3 and FIG. 4) which allows mutual fixation and fixation release for every predetermined pitch in the axial direction thereof, but details thereof will be described later.

The pressure portion cylinder 34 includes a tubular portion 44 which is coupled with the flexible tube 12 at an outlet port 43 of the liquid 19 at the distal end and concurrently, and which forms the pressure generating chamber 18 in the inside by a mechanism in which the hollow gasket 36 is inserted from an opening end 44a formed at the proximal end, and a pair of wings (first operation member) 46, 46 having flat plate shapes, which project from the outer circumferential surface of the tubular portion 44 toward the outer diameter direction and which are somewhat curved toward the distal side.

The tubular portion 44 has a taper region 44b in the shape of a circular truncated cone formed on the side more distal than the approximate center in the axial direction, and a cylindrical region 44c whose outer diameter and inner diameter are formed approximately uniformly and at the proximal end of which an opening end 44a is formed. The inner diameter of the cylinder region 44c is, for example, set at around 4mm to 16mm and the hollow gasket 36 axially slides in a liquid-tight state on the inner circumferential surface of aforesaid cylinder region 44c in the axial line direction. In this case, the inner diameter of the cylinder region 44c is allowed to be the same as or different from the inner diameter of the tube body 24 of the medicine ejection unit 14, but the inner volume of the pressure generating chamber 18 formed inside the tubular portion 44 is set equal to or greater than the inner volume of the action chamber 28 of the medicine ejection unit 14.

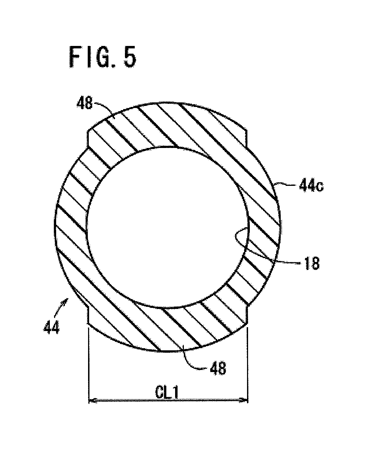

As shown in FIG. 3 and FIG. 5, on the outer circumferential surface at the proximal side of the tubular portion 44 (cylinder region 44c), there are formed a pair of first ribs 48, 48 composed of a shape which is formed by diameter-expanding & projecting a portion thereof toward the outer diameter direction. The first rib 48 is a thin plate-shaped member in the axial direction of the tubular portion 44 and a plurality of pieces (in case of this exemplified embodiment, 6 pieces) are installed in the axial direction by a pitch P1 in which the interval is approximately the same as or somewhat wider than the plate thickness thereof.

The hollow gasket 36 is formed, for example, by a flexible member such as rubber, silicone or the like, and as shown in FIG. 3, it includes an attachment portion 50 having a smaller diameter on the proximal side and a seal portion 52 having a larger diameter than that of the attachment portion 50. On the outer circumferential surface of the seal portion 52, there is provided a seal ring portion 52a in which a plurality of pieces are formed in parallel by being diameter-expanded toward the axial line direction. The outer diameter of the seal ring portion 52a (seal portion 52) is approximately the same as or somewhat larger than the inner diameter of the tubular portion 44 (cylinder region 44c). Thus, the seal ring portion 52a (seal portion 52) functions as a liquid-tight lip seal which is closely attached in a slidable manner with respect to the inner circumferential surface of the tubular portion 44.

In the hollow gasket 36, further, a slide hole 39 is penetratingly formed in the axial line direction, through which the rod 40a of the second pusher 40 passes slidably. On the inner circumferential surface of the slide hole 39, there is provided a seal ring portion 39a in which a plurality of pieces are formed in parallel by being diameter-reduced toward the axial line direction. The inner diameter of the seal ring portion 39a (slide hole 39) is approximately the same as or somewhat smaller than the outer diameter of the rod 40a of the second pusher 40. Thus, the seal ring portion 39a (slide hole 39) functions as a liquid-tight lip seal which is closely attached in a slidable manner with respect to the rod 40a.

As shown in FIG. 2 to FIG. 4, the first pusher 38 includes a first tubular portion 56 which is inserted into the inside of the tubular portion 44 (pressure generating chamber 18) by such a mechanism that the hollow gasket 36 is fixed at the distal end and a second tubular portion 58 forming a double tube structure by being provided coaxially by sandwiching a predetermined space toward the outer diameter direction of aforesaid first tubular portion 56, and the gap between these first tubular portion 56 and second tubular portion 58 functions as a tubular space 60 through which aforesaid tubular portion 44 (cylindical region 44c) passes.

As shown in FIG. 1 and FIG. 2, on the outer circumferential surface of the second tubular portion 58, there are formed a pair of finger hook portions 62, 62 which are in parallel in the axial line direction and project toward the outer diameter direction and which are in the shape of flat plates curved toward the distal side, and a rectangular ring shaped finger hook ring 64 which has a flat plate shape projecting from the approximately 180° opposite side of the finger hook portion 62 toward the outer diameter direction (see FIG. 1 and FIG. 2). The finger hook portion 62 and the finger hook ring 64 are regions to which the operator hooks his fingers together with the wing 46 of the pressure portion cylinder 34 when pulling-in the pressure portion cylinder 34 and the first pusher 38 so as to be close to each other.

As shown in FIG. 3 and FIG. 4, the first tubular portion 56 has an attachment hole 66 at its distal end thereof, to which a proximal end diameter-expanded portion of the attachment portion 50 of the hollow gasket 36 is fitted by insertion and fixed, and a guide hole 68 opened on the proximal side thereof.

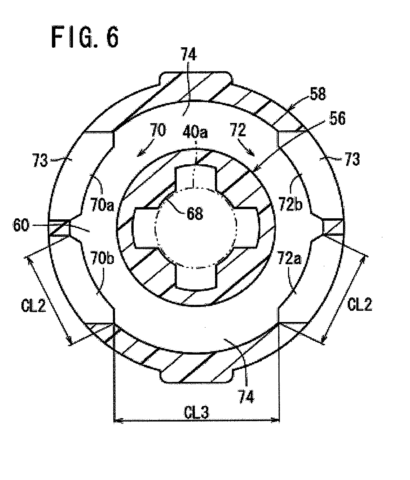

The guide hole 68 passes-through in the axial direction from the proximal end of the first tubular portion 56 to the attachment hole 66 of the distal end and thus, it has a function of allowing the rod 40a of the second pusher 40 to pass through to the slide hole 39 of the hollow gasket 36 and concurrently, of guiding the sliding in the axial direction thereof. As shown in FIG. 6, the guide hole 68 has a shape made by forming the center portion of the hole portion having an approximately cross-shape for the cross-section thereof as a circle along the axial line direction and guides the rod 40a slidably by the inner circumferential surfaces facing to 4 pieces of members having approximately trapezoid cross-sections, which are arranged at 90° intervals and which are elongated in the axial direction. In this manner, the guide hole 68 guides the rod 40a by 4-point support, so that it is possible to carry out the guide stably toward the axial line direction and moreover, it is possible to reduce the slide resistance with respect to the rod 40a appropriately. It is needless to say that it is allowed to constitute the guide hole 68 as a simple circle which slides on the full circumferential surface of the rod 40a.

As shown in FIG. 3 and FIG. 4, the second tubular portion 58 is constituted such that the proximal end thereof is closed by being coupled with the outer circumferential surface of the proximal end of the first tubular portion 56 and concurrently, the tubular portion 44 of the pressure portion cylinder 34 is insertable from the opened distal side to the inside of the tubular space 60. Therefore, the inner diameter of the second tubular portion 58 is set to be a diameter approximately the same as or somewhat larger than the maximum outer diameter (outer diameter of a pair of first ribs 48, 48) of the tubular portion 44.

As shown in FIG. 3, the length L1 in the axial direction from the distal end of the second tubular portion 58 to the distal end of the first tubular portion 56 is approximately same as the length L2 in the axial line direction of the attachment portion 50 of the hollow gasket 36 and therefore, as shown in FIG. 4, for the hollow gasket 36 fixed in the attachment hole 66 has a surface, the distal end surface thereof becomes approximately flush with the distal end surface of the second tubular portion 58.

As shown in FIG. 3 and FIG. 6, the second tubular portion 58 is formed with a pair of rib sets (second ribs) 70, 72 which are formed by shapes of diameter-reducing & projecting portions of the inner circumferential surface thereof toward the inner diameter direction and which are engageable with the first ribs 48 of the pressure portion cylinder 34. The rib sets 70, 72 are thin plate-shaped members in the axial line direction of the second tubular portion 58 and are installed by a plurality of numbers over the axial line direction of the second tubular portion 58 with a pitch P1 having an interval approximately same as or somewhat wider than the plate thickness thereof. More specifically, the rib sets 70, 72 are aligned toward the axial direction by a pitch P1 identical with that of the first ribs 48, the installation number of pieces thereof is more than that of the first ribs 48 and they are provided over the axial direction of the tubular space 60 so as to be engageable with each other even in a state in which the tubular portion 44 is to be inserted maximally into the inside of the tubular space 60.

As shown in FIG. 3 and FIG. 6, the respective ribs sets 70, 72 include first small ribs 70a, 72a and second small ribs 70b, 72b which are respectively aligned in the circumferential direction. For the first small ribs 70a, 72a and the second small ribs 70b, 72b, the length (width) CL2 in the circumferential direction thereof is formed as much as around half of the length CL1 in the circumferential direction of the first rib 48 (see FIG. 5), and the ribs constituting the respective rib sets 70, 72 each other (for example, first small rib 70a and second small rib 70b) are aligned by spacing a small gap. Also, the space CL3 in the circumferential direction between the rib set 70 and the rib set 72 is set to be somewhat larger than the length CL1 of the first rib and depending on this configuration, it is possible for the first rib 48 to pass through between the rib sets 70, 72 as a rib path 74 in the axial direction.

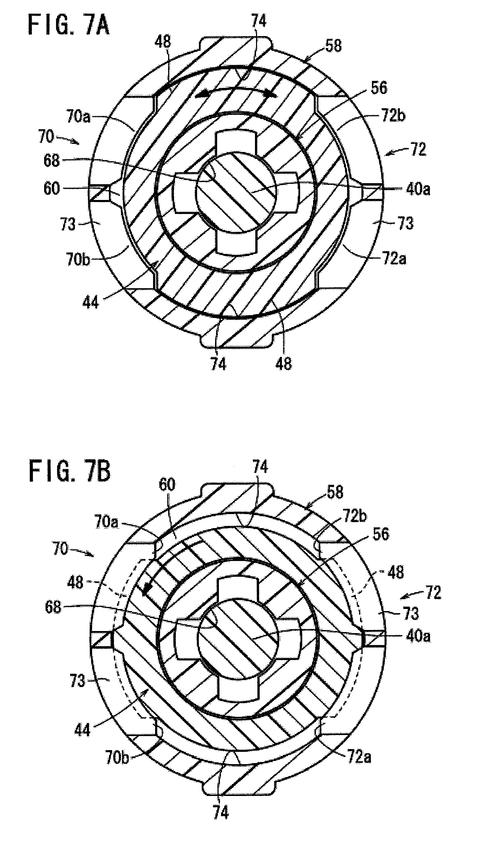

Therefore, by adjusting the rotation direction of the pressure portion cylinder 34 and by setting the position such that the first rib 48 corresponds to the rib path 74 as shown in FIG. 7A in a state in which the tubular portion 44 of the pressure portion cylinder 34 is inserted into the tubular space 60 of the first pusher 38 (see FIG. 4), the tubular portion 44 is moved smoothly in the axial direction of the tubular space 60, and it is possible, as shown by broken lines in FIG. 2, to slide the hollow gasket 36 inside the pressure generating chamber 18. On the other hand, as shown in FIG. 7B, by rotating the pressure portion cylinder 34 counterclockwise (or clockwise) as much as approximately 90° in which the respective first ribs 48 correspond to respective rib set 70, 72 and by engaging it between the rib sets 70 and between the rib sets 72 which are aligned in the axial direction, the positions of the pressure portion cylinder 34 and the first pusher 38 in the axial direction are fixed.

In this manner, the first ribs 48, rib sets 70, 72 (first small ribs 70a, 72a and second small ribs 70b, 72b) make both the pressure portion cylinder 34 and the first pusher 38 carry out fixation and fixation release for every predetermined pitch P1 in the axial direction thereof, and they function as an engagement mechanism 42 which changeably constitutes the fixation position in the axial direction thereof.

As understood from FIG. 3 and FIG. 6, at space portions in the axial direction of the respective first small ribs 70a, 72a and the respective second small ribs 70b, 72b aligning in the axial direction of the second tubular portion 58, there are provided hole portions 73 communicating with inner and outer surfaces of the second tubular portion 58. It is possible for these first small ribs 70a, 72a and the respective second small ribs 70b, 72b to be easily manufactured by means of a die having a structure in which protrusions provided in right and left split molds abut a die (center pin) for molding the guide hole 68, for example, when molding the first pusher 38 (second tubular portion 58) by injection mold, and the hole portions 73 are formed by aforesaid protrusions.

As shown in FIG. 1 to FIG. 4, the second pusher 40 has a long-scale and also narrow-diameter rod 40a and a press-in portion 40b which serves as a operation (steering) portion when aforesaid rod 40a is pushed into the pressure generating chamber 18 of the pressure portion cylinder 34. The outer diameter rod 40a is, for example, set at around 2mm to 6mm.

As shown in FIG. 3 and FIG. 4, at the distal end of the rod 40a, there is provided a distal end head portion 40c which is diameter-expanded in a flange shape and aforesaid distal end head portion 40c hooks on the distal side opening portion of the slide hole 39 of the hollow gasket 36, thereby preventing the pulling-out of the rod 40a from the hollow gasket 36. Note that, when the hollow gasket 36 is formed by a flexible member such as rubber or the like as described above, it is possible for the distal end head portion 40c having a somewhat larger diameter compared with that of the slide hole 39 (seal ring portion 39a) to be passed-through the inside of the slide hole 54 easily at the time of assembly.

With respect to such a medicine ejection unit 14 or the pressure generation unit 16, it is good if the tube body 24, the first pusher 38, the second pusher 40 or the like is formed by, for example, a polyolefin such as polypropylene, polyethylen, cyclic polyolefin, polymethylpentene 1 or the like, a resinous material such as polyester, nylon, polycarbonate, polyethersulphone or the like, or a metal material such as stainless steel or the like, and it is good if the floating gasket 26 or the hollow gasket 36 is formed by, for example, an olefin-based elastomer, a vulcanized rubber such as silicone rubber, butyl rubber, fluorine rubber or the like, and further, materials made by coating those with fluorine resin, or the like. When the flexible tube 12 has a constitution in which a thread of Kevlar, nylon, polyphenylene sulfide, stainless steel or the like is wound on the tube wall inside or the outer surface of the comparatively flexible polyolefin in a mesh or coil shape, it is suitable in terms of pressure resistance, but it is also allowed to employ a multi-layered tube in which polypropylene or fluorine resin having water resistance is arranged only on the inner surface.

These respective components are manufacturable by usual injection molding, extrusion molding, press molding or the like. Connection of the respective components is possible by adhesion by means of an adhesive agent or thermal fusion, and a typical method such as connection by means of a caulking tool, screw engagement or the like is possible to use for connection of the respective cylinders and the tube.

Next, it will be explained with respect to operations and actions of the medicine injection device 10 relating to the first exemplified embodiment constituted as above.

For example, in case of using the medicine injection device 10 in the percutaneous vertebroplasty, the inside the action chamber 28 of the medicine ejection unit 14 is filled with a predetermined amount (for example, 5ml) of the medicine (bone cement of CPC, PMMA or the like) beforehand. This filling is carried out by, for example, a method in which the first pusher 38 is pressed-in with respect to the second pusher 40 and in a state in which the hollow gasket 36 is pressed into the pressure generating chamber 18 to the maximum, a distal end tube, which is not shown, coupled to the ejection port 20 or the ejection port 20 is inserted into the container filled with bone cement which is medicine and the medicine is sucked inside the action chamber 28 by pulling the first pusher 38, a method in which a syringe, which is not shown, is coupled to the ejection port 20 and the syringe is pressurized to fill the inside of the action chamber 28 with the medicine. Next, the ejection port 20 of the medicine ejection unit 14 is installed with an ejection rod (needle for bone cement) or the like which is not shown and the filling of the medicine is executed under the X-ray illumination.

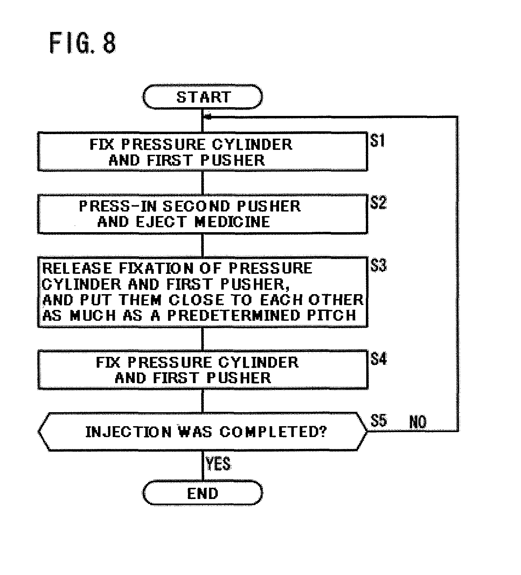

Operation procedure relating to the medicine ejection by the medicine injection device 10 is shown in FIG. 8.

In order to eject the medicine inside of the action chamber 28 from the ejection port 20, first, both the pressure portion cylinder 34 and the first pusher 38 are fixed in an appropriate initial position corresponding to the amount of injection of the medicine (for example, 5ml) by the first rib 48 and the rib sets 70, 72 of the engagement mechanism 42 (step S1) Next, the press-in portion 40b is pressed-in toward the distal end direction by a palm or the like from a state in which the second pusher 40 is pulled to the proximal side maximally (see FIG. 2) so as to make the rod 40a progress, and as shown with a broken line in FIG. 4, the distal end head portion 40c is pressed into the pressure generating chamber 18 (step S2). More specifically, both the pressure portion cylinder 34 and the first pusher 38 are fixed in the axial direction and the hollow gasket 36 is also fixed by the first pusher 38, so that the rod 40a is pressed into the pressure generating chamber 18 while sliding in the slide hole 39 of the hollow gasket 36.

Thus, a pressure corresponding to the void inner volume of the liquid 19 as much as the progress inside the pressure generating chamber 18 of the rod 40a is generated inside the pressure generating chamber 18 and concurrently, aforesaid pressure causes the liquid 19 composed of an incompressible fluid (for example, water) filling the insides of the pressure generating chamber 18 and the flexible tube 12 to flow from the flow-in port 31 into the working chamber 30 and thus, the floating gasket 26 is pressed from the working chamber 30 side to the action chamber 28 side. At that time, aforesaid liquid 19 is substantially not compressed and moreover, the outer diameter (for example, 5mm) of the rod 40a of the second pusher 40 is narrow-diameter, so that it is possible to generate a high pressure of 100 atmospheres (about 100Mpa) or more in the inside of the chamber 18 easily even by pushing with a human hand. Therefore, the high pressure generated in the pressure generating chamber 18 is not absorbed by the liquid 19 and acts upon the floating gasket 26 directly and more specifically, the floating gasket 26 progresses as much as the void inner volume of the rod 40a and the appropriate amount (for example, 1ml) of the medicine corresponding to as much as aforesaid void inner volume is ejected from the ejection port 20 reliably under the high pressure. In other words, by sealing aforesaid liquid 19 in the working chamber 30 or the like, aforesaid liquid 19 functions as a piston rod for coupling the rod 40a with the floating gasket 26 approximately mechanically and the press-in pressure by the second pusher 40 acts upon ejection of the medicine directly.

Subsequently, the pressure portion cylinder 34 is rotationally steered with respect to the first pusher 38 by steering the wing 46 and a engagement state between the first rib 48 and the rib sets 70, 72, which constitute the engagement mechanism 42, is thereby released (see FIG. 7A), and the first rib 48 is moved through the rib path 74. Thus, the pressure portion cylinder 34 and the first pusher 38 are made to approach each other, for example, by as much as one pitch or as much as few pitches based on the pitch P1 of the first rib 48 and the rib sets 70, 72, more specifically, as much as the void inner volume by the second pusher 40 (step S3).

In that case, the hollow gasket 36 (first tubular portion 56) progresses into the pressure generating chamber 18, so that the liquid 19 is pushed in aforesaid pressure generating chamber 18 and a certain degree of pressure is generated, but the diameter (for example, 12mm) of the hollow gasket 36 is considerably large compared with the diameter (for example, 5mm) of the rod 40a and more specifically, it is difficult to generate the high pressure such a value of the rod 40a depending on a human hand, so that the floating gasket 26 also does not progress and ultimately, a force acts toward the direction in which the rod 40a of the second pusher 40 is returned to the original position from the liquid 19 of the pressure generating chamber 18 and the second pusher retreats as much as the volume corresponding to the press-in amount in step S2 until the distal end head portion 40c abuts onto the opening end of the slide hole 39 of the hollow gasket 36. Consequently, it becomes in a state in which the whole pressure generated by the progress of the second pusher 40 and the hollow gasket 36 is released. More specifically, the generating pressure as much as the void inner volume of the second pusher 40 is used for ejection of the medicine and thus, it is possible to eject the appropriate amount (for example, 1ml) at the high pressure and moreover, if pushing the second pusher 40 is stopped after ejection, the pressure inside the system is released reliably.

Therefore, again, by fixing the pressure portion cylinder 34 and the first pusher 38 by the engagement mechanism 42 (step S4), the hollow gasket 36 progresses as much as a predetermined pitch (as much as void inner volume by second pusher 40) of the engagement mechanism 42 toward the pressure generating chamber 18 side and the floating gasket 26 also progresses as much as the volume corresponding to the void inner volume of the second pusher 40 of the hollow gasket 36 and other than that, it becomes in an approximately similar state as the initial state before the injection start shown in FIG. 2.

Therefore, in step S5, the operations of step 1 to step 4 described above are repeated until a necessary and sufficient amount of the medicine is injected inside the bone (NO in step S5), and after the injection of the necessary and sufficient amount of the medicine is completed (YES in step S5), the ejection port 20 of the medicine ejection unit 14 is removed to the outside of the body, thereby making it possible to execute the percutaneous vertebroplasty easily.

In this case, in the medicine injection device 10 relating to this exemplified embodiment, it is possible to eject a constant amount (for example, 1ml) of the medicine for every one time of ejection process (step S1 to S4) under a predetermined high pressure reliably and moreover, the pressure inside the system of the medicine injection device 10 is released in the time interval which intervenes until the next ejection process. In other words, by the fact that the medicine injection device 10 is constituted as a two-stage piston type injection device provided with the second pusher 40 and the hollow gasket 36, the pressure inside the system is released at the moment of releasing the hand after the second pusher 40 is pressed-in and thereafter, it does not happen that the pressure generates also in a case in which the hollow gasket 36 is made to progress.

Thus, for example, it is possible to reliably prevent a high pressure state of around 100 atmospheres from acting on the flexible tube 12, the floating gasket 26 and the like on a steady basis and it is possible to prevent erroneous medicine ejection or the like during the operation standby or the like and prevent the load at the connection portion with the flexible tube 12 or the like. Also, for example, even in a case in which the injection at 4ml into the inside of the bone becomes what is necessary and sufficient despite a previous plan to inject the medicine of 5ml (5 times ejection processes at 1ml) into the inside of the bone, pressure is not generated on the liquid 19 inside aforesaid medicine injection device 10 at the time point when the forth round of the ejection process is finished, so that the medicine is not erroneously ejected from the ejection port 20 and it is possible to remove the medicine ejection unit 14 from the patient.

It is possible for the pressure portion cylinder 34 and the first pusher 38 to adjust the coupling position in the axial direction each other for every the pitch P1 of the first rib 48 and the rib sets 70, 72 easily only by a simple operation in which they are rotated each other and are shifted in the axial direction and moreover, the second pusher 40 is returned to the initial position reliably at the time of aforesaid adjustment. Consequently, after ejection of the medicine by the second pusher 40, it is possible to set the pressure portion cylinder 34, the first pusher 38 and the second pusher 40 to the position (initial position) correspond to the next ejection process.

In the medicine injection device 10, only one piece of the hollow gasket 36 is enough for the gasket which slides at the pressure generation unit 16 and also the engagement mechanism 42 between the pressure portion cylinder 34 and the first pusher 38 is a constitution in which only the ribs are provided. Further, the second pusher 40 enables pressure to be generated by only being passed through the inside of the first pusher 38 and pressed-in, and after ejection of the medicine, the second pusher 40 is returned to the initial position by only moving the pressure portion cylinder 34 and the first pusher 38. In this manner, with respect to the medicine injection device 10, the structure thereof is simple and also low-cost and concurrently, the injection operation is constituted simply.

Note that it is necessary for aforesaid pitch P1 which serves as the standard of the movement amount of the hollow gasket 36 after the ejection to be set such that as much as the maximum void inner volume by the second pusher 40 is absorbable, in other words, it is necessary to be able to fix the pressure portion cylinder 34 and the first pusher 38 by making the hollow gasket 36 progress for just as much as the void inner volume by the second pusher 40. Therefore, the void inner volume V1 corresponding to 1 pitch of the pitch P1 of the hollow gasket 36 is set within 1 time or preferably around 0.2 times to 1 time (V1 = 0.2V2 to V2) with respect to the maximum void inner volume V2 pressable by the second pusher 40 in a state in which the pressure portion cylinder 34 and the first pusher 38 are fixed. Thus, after the pressing-in of the second pusher 40, it becomes possible for the pressure portion cylinder 34 and the first pusher 38 to approach each other by at least as much as one pitch reliably.

Also, in a case in which the medicine injection device 10 is used for a medicine injectable at a certain degree of low pressure and for the space to be injected, the pressure portion cylinder 34 and the first pusher 38 are set to a state in which the first rib 48 corresponds to the rib path 74 and in this state, the pressure portion cylinder 34 and the first pusher 38 are pulled toward each other. In that case, a large amount of injection at one time by the hollow gasket 36 becomes possible compared with high-pressure injection in small amounts by the second pusher 40 (see broken line in FIG. 2). More specifically, in the medicine injection device 10, it is a two-stage piston (two-stage plunger) structure by the first pusher 38 (hollow gasket 36) and the second pusher 40 and only the hollow gasket 36 which is one of those pistons is used and if the second pusher 40 which is the other piston is fixed while being kept at the initial position, approximately similarly to a usual syringe or the like, it is possible to use it for the medicine ejection in a large amount and also at low pressure, and versatility is high. It is possible for such a use method approximately similar to a usual syringe or the like to be used effectively even when a predetermined medicine from the ejection port 20 is made to fill (sucked into) the inside of the action chamber 28 as described above.

In the medicine injection device 10, by providing the wing 46 of the pressure portion cylinder 34, and the finger hook portions 62 and the finger hook ring 64 of the first pusher 38, thereby grasping both with a single hand (or both hands), it is possible to rotate or pull-in the pressure portion cylinder 34 and the first pusher 38 easily and steerability is high. Also, in the medicine injection device 10, the pressure portion cylinder 34 is inserted into the first pusher 38 to the maximum and by pressing-in the second pusher 40 to the maximum, it is compactly storable.

The medicine ejection unit 14 is connected with the pressure generation unit 16, which lies on the operation side, through the long scale flexible tube 12, so that it is possible to carry out the injection operation at a position apart from the medicine ejection unit 14 near to the X-ray tube. Therefore, if the length of the flexible tube is, for example, to be 5cm or more, the operator's exposure to X-rays on an occasion of injection of the bone cement can be reduced, but it is preferable for the length to be 15cm or more to 50cm or less. Note that depending on the use condition or the like of the medicine injection device 10, it is also possible to omit the flexible tube 12 and to employ a constitution in which the medicine ejection unit 14 is provided at the distal end of the pressure portion cylinder 34 in direct connection or integrally.

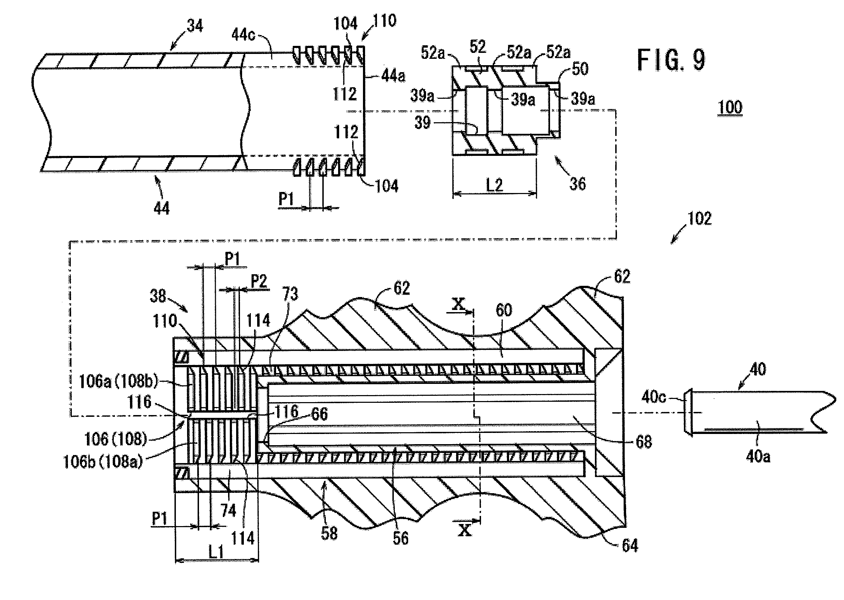

FIG. 9 an exploded side cross-sectional view in which the main portion of a pressure generation unit 102 constituting a medicine injection device 100 relating to a second exemplified embodiment of the present invention is magnified. In the medicine injection device 100 relating to this second exemplified embodiment, elements which are the same as or similar to the elements of the medicine injection device 10 relating to the first exemplified embodiment described above are given the same reference numerals for exerting the same or similar functions and effects and detailed explanation will be omitted.

The medicine injection device 100 is provided with a pressure generation unit 102 in which the constitution of the pressure generation unit 16 of the medicine injection device 10 described above is changed. As shown in FIG. 9, the pressure generation unit 102 is basically similar to the pressure generation unit 16 shown in FIG. 3 or the like except for inclusion of an engagement mechanism 110 constituted by a first rib 104 and rib sets 106, 108 instead of the engagement mechanism 42.

As shown in FIG. 9, the first rib 104 is different from the first rib 48 shown in FIG. 3 or the like in that an inclination surface (first inclination surface) 112 whose width narrows toward the proximal side in the axial direction of the tubular portion 44 is formed at both end portions in the circumferential direction (longitudinal direction) thereof.

The rib sets 106, 108 include, similar to the rib sets 70, 72 shown in FIG. 3 or the like, first small ribs 106a, 108a and second small ribs 106b, 108b, which are respectively aligned in the circumferential direction, but there is a difference from aforesaid rib set 70 (72) in that the positions of the first small rib 106a and the second small rib 106b (first small rib 108a and second small rib 108b) constituting each rib set 106 (108) in the axial direction of the second tubular portion 58 deviate from each other.

More specifically, in the axial direction of the second tubular portion 58, the first small ribs 106a alone and the first small ribs 108a alone (second small ribs 106b alone and second small ribs 108b alone are also similar) are aligned at the same pitch P1 as that of the first ribs 104, aforesaid rib sets 70 or the like, but the first small rib 106a and the second small rib 106b (first small rib 108a and second small rib 108b are also similar) deviate from each other by as much as a pitch P2 which is half the pitch P1. In other words, in the rib sets 106, 108, with respect to the first small rib 106a and the first small rib 108a (second small rib 106b and second small rib 108b) which face to each other, the phases thereof in the axial direction of the second tubular portion 58 are the same, and with respect to the first small rib 106a and the second small rib 106b (first small rib 108a and second small rib 108b) which adjoin each other in the circumferential direction, the phases thereof in aforesaid axial direction deviate from each other by as much as a half pitch.

At the end portion facing the rib path 74 of such a rib set 106 (108), more specifically, at the end portions on the sides facing the rib paths 74 of the first small ribs 106a, 108a and the second small ribs 106b, 108b, there are formed inclination surfaces (second inclination surfaces) 114 whose widths narrow toward the distal side in the axial direction of the second tubular portion 58. The inclination surface 114 has a shape that the inclination surface 112 of the first rib 104 can get over while they slide on each other.

Therefore, in the medicine injection device 100 relating to this exemplified embodiment, for example, on an occasion when the second pusher 40 is pressed into the pressure generating chamber 18 and the medicine is ejected from the ejection port 20 and thereafter, the hollow gasket 36 is made to progresses inside the pressure generating chamber 18 by making the pressure portion cylinder 34 and the first pusher 38 close to each other, the pressure portion cylinder 34 and the first pusher 38 are rotated in opposite directions as if they are twisted oppositely to each other. In that case, it is possible for the pressure portion cylinder 34 and the first pusher 38 to approach each other by every aforesaid pitch P2 while yielding a sequential switch from a state in which the first rib 104 is engaged with the first small ribs 106a, 108a to a state in which it corresponds to the rib path 74 and further, to a state in which it is engaged with the second small ribs 106b, 108b.

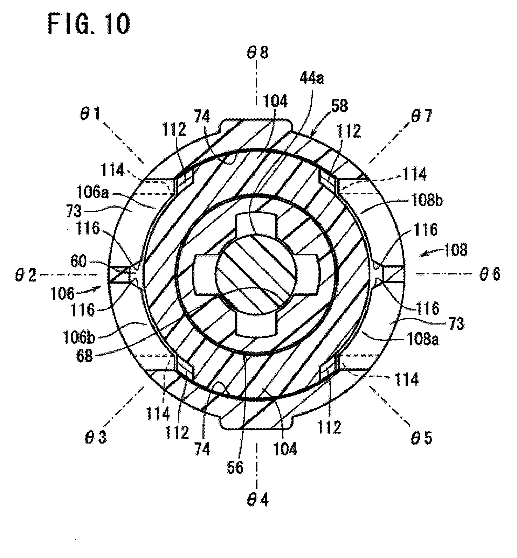

Then, next, it will be explained further specifically with respect to the engagement operation and the movement operation between the pressure portion cylinder 34 and the first pusher 38 by the engagement mechanism 110 constituting the medicine injection device 100 with reference to FIG. 10 to FIG. 12.

FIG. 10 shows a cross-sectional view at a position along an X-X line in FIG. 9 in a state in which the pressure portion cylinder 34 is inserted in the tubular space 60 of the first pusher 38. In FIG. 10 to FIG. 12, for convenience of explanation, the tubular space 60 of the first pusher 38, into which the pressure portion cylinder 34 is inserted, is divided into eight in the circumferential direction and angles from θ1 to θ8 are attached thereto. More specifically, in FIG. 10 to FIG. 12, the second tubular portion 58 is shown by being fixed in the rotation direction, and for example, the first small rib 106a is arranged between angles from θ1 to θ2, the first small rib 106b is arranged between angles from θ2 to θ3 and the rib path 74 is arranged between angles from θ7 to θ1.

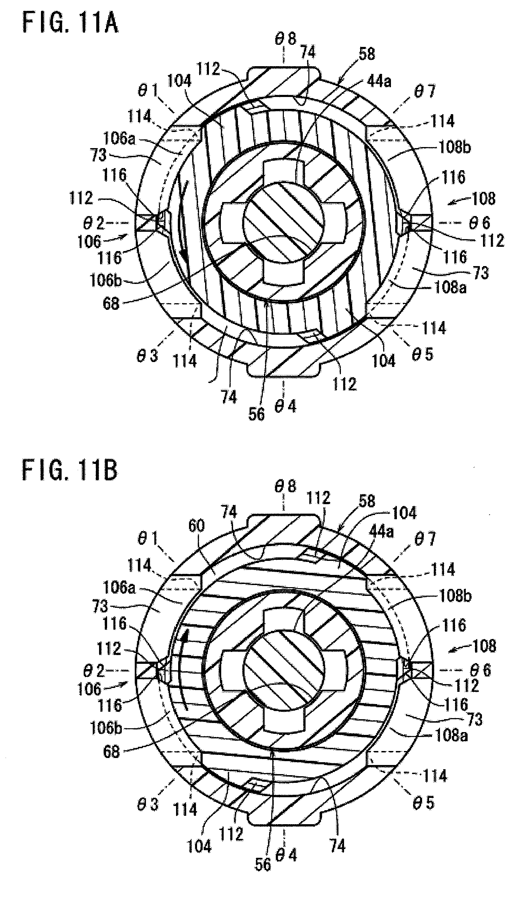

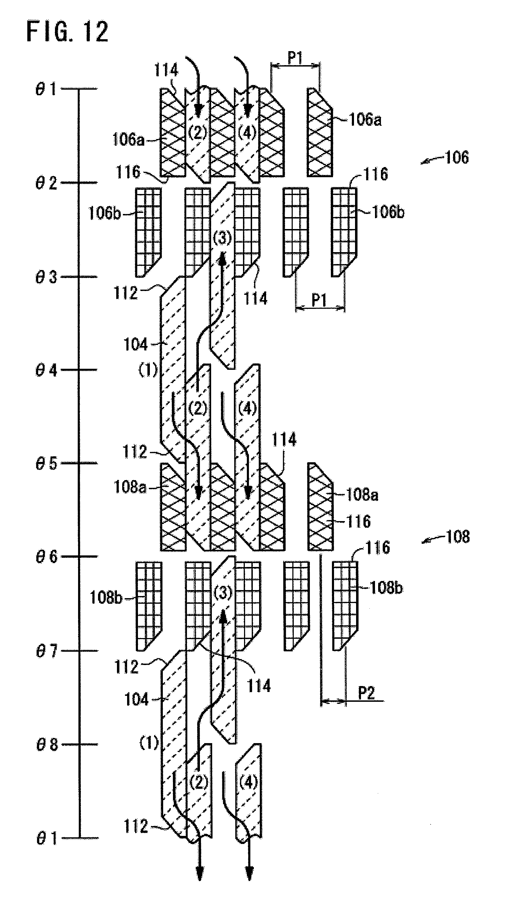

First, the first rib 104 (pressure portion cylinder 34) is rotated counterclockwise as shown in FIG. 11A from a state in which the first rib 104 lies in the position corresponding to the rib path 74 (position (1) of FIG. 12). In that case, the respective inclination surfaces 112, 112 of the respective first ribs 104 get over the inclination surfaces 114, 114 of the second small ribs 106a, 108a of the respective rib sets 106, 108 respectively and thereafter, come into contact with the stopper end portions 116 which lie on the inside of the rib sets 106, 108 of the first small ribs 106b, 108b and which do not have the inclination surface 114, and the furthermore rotation of the pressure portion cylinder is stopped. As a result thereof, the first ribs 104 move from the position (1) to the position (2) in FIG. 12 and stop and concurrently, the pressure portion cylinder 34 and the first pusher 38 are approached to each other as much as the pitch P2 and engaged with each other, and fixed in the axial direction. More specifically, the respective first ribs 104 are arranged between angles from θ4 to θ6 and between angles from θ8 to θ2 respectively and the first ribs 104 are engaged with the second small ribs 106a, 108a.

Subsequently, the first ribs 104 (pressure portion cylinder 34) are rotated clockwise as shown in FIG. 11B from a state in which the first ribs 104 lie in the position (2) in FIG. 12 in this manner. In that case, the respective inclination surfaces 112, 112 of the respective first ribs 104 move over the inclination surfaces 114, 114 of the second small ribs 106b, 108b of the respective rib sets 106, 108 respectively and thereafter, come into contact with the stopper end portions 116 of the first small ribs 106a, 108a, and the furthermore rotation of the pressure portion cylinder is stopped. As a result thereof, the first ribs 104 move from the position (2) to the position (3) in FIG. 12 and stop and concurrently, the pressure portion cylinder 34 and the first pusher 38 are made to approach each other by as much as the pitch P2, engaged with each other and fixed in the axial line direction. More specifically, the respective first ribs 104 are arranged between angles from θ2 to θ4 and between angles from θ6 to θ8 respectively and the first ribs 104 are engaged with the second small ribs 106b, 108b.

Subsequently, in a case in which the first ribs 104 are rotated counterclockwise again from a state in which the first ribs 104 lie in the position (3) in FIG. 12, the first ribs 104 are set to be in the position shown in FIG. 11A (position (4) in FIG. 12) again and are engaged with the first small ribs 106a, 108a and thereafter, by rotating the pressure portion cylinder 34 and first pusher 38 alternately clockwise and counterclockwise with each other, it is possible to fix the pressure portion cylinder 34 and the first pusher 38 while approaching each other gradually in the axial direction.

Such as the above, according to the medicine injection device 100 relating to this exemplified embodiment, in addition to the functional effect of the medicine injection device 10 relating to the first exemplified embodiment described above and further, by rotating the pressure portion cylinder 34 and the first pusher 38 alternately clockwise and counterclockwise for every approximately 180°, it is possible to fix the pressure portion cylinder 34 and the first pusher 38 while approaching each other for every the pitch P2 which is a half of the pitch P1. Consequently, it is possible to make the hollow gasket 36 progress in the inside of the pressure generating chamber 18 by pulling-in the pressure portion cylinder 34 and the first pusher 38 easily and also reliably by a sensory operation depending on only the fingers, without considering the positional relation between the first ribs 104 and the rib sets 106, 108.

It is needless to say also with respect to the medicine injection device 100 that it is set to be in a state in which the first ribs 104 are made to correspond to the rib path 74, and in this state, by pulling-in the pressure portion cylinder 34 and the first pusher 38 toward each other, it is possible to use such a use method by which the medicine is ejected at one time similarly to that of a usual syringe or the like.

The present invention is not only limited by the exemplified embodiments mentioned above and it is needless to say that various constitutions or processes can be employed without departing from the gist of the present invention.

For example, it is allowed for the first ribs 48, 104 or the rib sets 70, 72, 106, 108 which are the second ribs to be provided by two pairs or more in the circumferential direction, and also to be employed by only one piece, by three pieces or more without providing one pair in the circumferential direction.

With respect to the first ribs 48, 104 or the rib sets 70, 72, 106, 108, it is allowed for each one side thereof to employ only one piece in the axial direction of the tubular portion 44, but in consideration of the engagement strength between the pressure portion cylinder 34 and the first pusher 38, stability on an occasion of the engagement and the like, it is preferable that both are installed by a plurality of pieces.

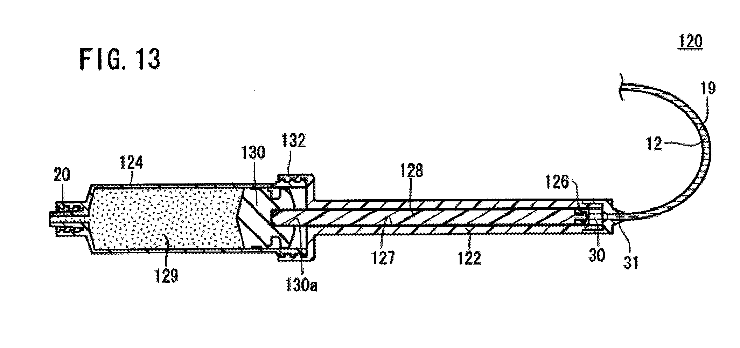

Also, it is allowed for the constitution of the medicine ejection unit 14 to be a constitution other than those shown in FIG. 1 and the like and in brief, it is enough if it is a constitution capable of appropriately ejecting the medicine from the medicine ejection port by a pressure action from the pressure generation unit 16, and for example, it is also possible to employ the constitution shown in FIG. 13.

FIG. 13 is a partially omitted cross-sectional side view showing a medicine ejection unit 120 relating to a modification example. As shown in FIG. 13, the medicine ejection unit 120 is provided with a first tube body 122 and a second tube body 124 instead of the tube body 24 compared with the medicine ejection unit 14 shown in FIG. 1 and FIG. 2.

With respect to the first tube body (tube body) 122, the flow-in port 31 of the liquid 19 at the distal end thereof is connected with the proximal end of the flexible tube 12 and concurrently, the distal side of a gasket (sub gasket) 126 forming the working chamber 30 is coupled to a rod 128. The rod 128 is inside an action chamber 127, is formed hard by a similar material to that of the second pusher 40 or the like, and is coupled to the gasket 126 by insert molding or the like.

With respect to the second tube body (medicine container) 124, the ejection port 20 is provided at the distal end thereof and concurrently, a medicine chamber 129 in which the medicine (bone cement) is accumulated is formed between the ejection port 20 and a cap compatible gasket 130. The proximal end of the second tube body 124 is constituted detachably at a skirt portion (medicine container connection portion) 132 of the distal end of the first tube body 122 and more specifically, regarding the second tube body 124, with the cap compatible gasket 130 functioning as a cap, it functions as a replaceable medicine container (medicine cartridge) with respect to the first tube body 122. The cap compatible gasket 130 includes an attachment hole 130a opened on the proximal side, and when the second tube body 124 is coupled to the first tube body 122, the distal end of the rod 128 is inserted into the attachment hole 130a and it is coupled to aforesaid rod 128.

Also with respect to such a medicine ejection unit 120, it is a unit which operates approximately similarly to the medicine ejection unit 14, and when the pressure from the pressure generation unit 16 acts upon the working chamber 30 of the first tube body 122, the rod 128 to which the gasket 126 is coupled progresses, the cap compatible gasket 130 connected to the rod 128 progresses inside the second tube body 124, whereby it is possible to eject the medicine inside the medicine chamber 129 from the ejection port 20. More specifically, the gasket 126 which functions as the sub gasket relating to the medicine ejection and the cap compatible gasket 130 are coupled by the rod 128, thereby functioning integrally as a plunger enterable into the second tube body 124 which is connected on the medicine chamber 129 side, and thus it is possible to eject the medicine by the pressure which acts through the liquid 19 from the pressure generation unit 16 side. By using such a medicine ejection unit 120, it is possible to easily replace and use a medicine container of a kind or a volume, which is appropriate for the kind of medicine to be ejected or the volume of a space to be injected and it is possible to further improve versatility and usability of aforesaid medicine injection device 10 (100).

## Claims

1. A medicine injection device provided with a medicine ejection unit (14, 120) including a flow-in port (31) of fluid (19) on the proximal side of a tube body (24, 122) in which the inside of the tube body (24, 122) is partitioned by a sub gasket (26, 126) slidable in a liquid-tight state into a working chamber (30) on the flow-in side of the fluid (19) and an action chamber (28, 127) on the distal side, and a pressure generation unit (16) including a pressure generating chamber (18) which generates pressure acting upon the working chamber (30) through the fluid (19) in which medicine is ejected by making the sub gasket (26, 126) progress by the pressure, **characterized in that**
the pressure generation unit (16) comprises:
a pressure portion cylinder (34) provided inside the pressure generating chamber (18),
a first pusher (38) provided with a main gasket (36) for defining the inner volume of the pressure generating chamber (18) by sliding inside the pressure portion cylinder (34) in a liquid-tight state, and
a second pusher (40) which passes-through the inside of the first pusher (38) and concurrently, which is slidable in a liquid-tight state in a slide hole (39) penetratingly formed in the slide direction of the main gasket (36) and which generates the pressure inside the pressure generating chamber (18) by pressing a distal end head portion (40c) into the pressure generating chamber (18); and
the pressure portion cylinder (34) and the first pusher (38) are coupled through an engagement mechanism (42, 110) in which fixation and fixation release are possible for each other for every predetermined pitch in the axial line direction thereof.

2. The medicine injection device according to claim 1, **characterized in that** the region from the working chamber (30) to the pressure generating chamber (18) is filled with an incompressible fluid (19) which is substantially not compressed by the pressure generated in the pressure generation unit (16).

3. The medicine injection device according to claim 1 or 2, **characterized in that**
the pressure portion cylinder (34) communicates with the working chamber (30) at the distal side thereof and concurrently, includes, at the proximal side thereof, a tubular portion (44) formed with an opening end (44a) through which the main gasket (36) is inserted; and
the first pusher (38) includes a first tubular portion (56) on which the main gasket (36) is fixed at the distal side thereof and which is insertable into the tubular portion (44), and a second tubular portion (58) forming a tubular space (60) into which the tubular portion (44) is insertable with respect to the first tubular portion (56) by being provided coaxially by sandwiching a predetermined space in the outer diameter direction of the first tubular portion (56).

4. The medicine injection device according to claim 3, **characterized in that**
the engagement mechanism (42, 110) includes:
a first rib (48, 104) which is provided on the outer circumferential surface of the tubular portion (44) of the pressure portion cylinder (34) and which projects from a portion in the circumferential direction of the outer circumferential surface toward the outer diameter direction, and
second ribs (70, 72, 106, 108) which are provided by a plurality of numbers and by having a predetermined pitch in the axial line direction of the second tubular portion (58) on an inner circumferential surface of the second tubular portion (58) of the first pusher (38) and which are engageable with the first rib (48, 104) for every pitch of the predetermined pitch in the axial line direction of the second tubular portion (58) by projecting from the portion in the circumferential direction of the inner circumferential surface toward the tubular space (60); and
there is formed, in the tubular space (60), a rib path (74) in which the first rib (48, 104) is passable on the side of the second rib (70, 72, 106, 108) toward the axial line direction of the second tubular portion (58).

5. The medicine injection device according to claim 4, **characterized in that**
the first rib (48, 104) is a plate-shaped member which is diameter-expanded for a portion of the outer circumferential surface of the tubular portion (44) toward the outer diameter direction thereof and concurrently, is provided by at least one pair facing to each other in the diameter direction of the tubular portion (44);
the second rib (70, 72, 106, 108) is a plate-shaped member which is diameter-reduced for a portion of the inner circumferential surface of the second tubular portion (58) toward the inner diameter direction thereof and concurrently, is provided by at least one pair facing to each other in the diameter direction of the second tubular portion (58); and
the rib path (74) is formed between the facing second ribs (70, 72, 106, 108).

6. The medicine injection device according to claim 5, **characterized in that**
there is provided, for the second rib (106, 108), a rib set (106, 108) by one pair facing to each other in the diameter direction of the second tubular portion (58) in which a first small rib (106a, 108a) and a second small rib (106b, 108b) which are formed to be shorter than the circumferential direction length of the first rib (104) are aligned in the circumferential direction of the inner circumferential surface of the second tubular portion (58);
the first small ribs (106a, 108a) alone and the second small ribs (106b, 108b) alone are respectively arranged to face each other between the facing rib sets (106, 108); and
in the axial line direction of the second tubular portion (58), the pitch between the first small ribs (106a, 108a) alone and the pitch between the second small ribs (106b, 108b) alone are respectively set to be the predetermined pitches with which the first rib (104) is engageable, and the pitches between the first small ribs (106a, 108a) and the second small ribs (106b, 108b) are set to be half the predetermined pitches.

7. The medicine injection device according to claim 6, **characterized in that**
there is formed, at both end portions of the first rib (104) in the circumferential direction of the tubular portion (44), a first inclination surface (112) whose width narrows toward the proximal side in the axial line direction of the tubular portion (44); and
there is formed, at an end portion facing to the rib path (74) of the first small rib (106a, 108a) and the second small rib (106b, 108b) which constitute the rib set (106, 108), a second inclination surface (114) whose width narrows toward the distal side in the axial line direction of the second tubular portion (58) and which the first inclination surface (112) can get over by sliding.

8. The medicine injection device according to claim 4, **characterized in that**
the first rib (48, 104) has a predetermined pitch and is provided by a plurality of numbers in the axial line direction of the second tubular portion (58), and the respective first ribs (48, 104) are engageable with the respective second ribs (70, 72, 106, 108) simultaneously.

9. The medicine injection device according to claim 1, **characterized by** comprising
a medicine ejection port (20) in the action chamber (28) of the tube body (24).

10. The medicine injection device according to claim 1, **characterized in that**
there is included a medicine container connection portion (132) at the distal end portion of the tube body (122), and the sub gasket (126) includes a plunger (128, 130) enterable into the inside of a medicine container (124) which is connected to the action chamber (127) side.

11. The medicine injection device according to claim 2, **characterized in that**
there is included a flexible tube (12) which makes communication between the working chamber (30) of the medicine ejection unit (14) and the pressure generating chamber (18) of the pressure generation unit (16), and
the insides of the working chamber (30), the pressure generating chamber (18) and the flexible tube (12) are filled with the incompressible fluid (19).

12. The medicine injection device according to claim 1, **characterized in that**
the medicine is bone cement to be injected into the inside of the bone.
